# EUROPEAN PATENT APPLICATION

(11) **EP 1 396 498 A1**
(43) Date of publication of application: **10.03.2004**
(21) Application number: 02019756.2
(22) Date of filing: 04.09.2002
(51) Int. Cl.: C07K 14/395, C12N 15/31, C07K 17/00, G01N 33/68, C07K 1/22

(54) **A pair of polypeptides with specific and strong interaction**

(71) Applicant: Max-Planck-Gesellschaft zur Förderung der Wissenschaften e.V., 80539 München (DE)
(72) Inventor: Andag, Uwe, 37115 Duderstadt (DE); Schmitt, Hans Dieter, 37077 Göttingen (DE)
(74) Representative: Wichmann, Hendrik, Dr.

(57) **Abstract**

The present invention relates to non-natural polypeptides selected from a pair of polypeptides which can bind to one another in the presence of high salt. Furthermore, the invention relates to nucleic acidc encoding these polypeptides, an affinity matrix comprising the non-natural polypeptides of the invention and a solid support, and a kit comprising an affinity matrix of the invention and a container. In addition, the invention relates to the use of a first polypeptide of the invention for the detection of a second polypeptide of the invention and also the use of a peptide derived from Dsl1p and a peptide derived from δ-COP for the dimerization of polypeptides. Finally, the invention provides a method for the purification of a polypeptide of the invention

## Description

### BACKGROUND OF THE INVENTION

The structural integrity of membrane-bound organelles requires the recycling of lipids and polypeptides. Between Golgi and ER this retrograde transport is mediated by COPI-coated vesicles. The COPI-coat from yeast and mammals consists of seven COP polypeptides (α, β', β, γ, δ, ε, and ζ-COP = coatomer) and the small GTPase ARF1.

Two classes of accessory polypeptides are important for the coordinated interplay of tethering and docking of vesicles to their target membrane. The ATPase NSF together with its cofactor α-SNAP, which in yeast are encoded by the *SEC18* and *SEC17* genes. NSF/Sec18p can disassemble *cis*-SNARE complexes formed during previous fusion events and thereby activate the SNAREs for a new round of vesicle docking. The second class of polypeptide is a compartmentally specialized member of the family of Ypt/Rab GTPases (Nichols and Pelham, Biochim Biophys Acta (1998): 1404(1-2):9-31; Gotte et al., Subcell Biochem. (2000): 34:133-73).

In addition to coatomer and ARF1, Golgi-ER retrograde transport in yeast requires the ARF1 nucleotide exchange factors Gea1/2p, the ARF GTPase activating polypeptides Gcs1p and Glo3p and the NSF homologue Sec18p. The SNARE or SNARE-like polypeptides involved in fusion at the ER are Ufelp, Sec22p, Bet1p and Sec20p. Tip20p as a Sec20p-interacting protein is also essential for retrograde transport. So far no evidence for an involvement of a Ypt/Rab-like GTPase or specific tethering factors was provided (Harter and Reinhard, Subcell Biochem (2000): 34:1-38).

Recently, a new coatomer interacting factor, Dsl1p, which is essential for retrieval of proteins from Golgi to ER, has been identified (VanRheenen et al., 2001; Andag et al., 2001; Reilly et al., 2001). Dsl1p might interact with Tip20p (Ito et al., 2001; Reilly et al., 2001) and δ-COP (Reilly et al., 2001). However, Reilly et al. have only shown the putative interaction between Dsl1p and δ-COP in a two-hybrid assay and two-hybrid assays are not able to determine, whether a putative protein-protein-interaction is direct or mediated by an unknown bridging factor, be it another protein or even an RNA.

Andag et al. (2001) have shown an interaction between Dsl1 and the coatomer complex by immunprecipitation. However, the coatomer-Dsl1p-interaction could have been based on an interaction between Dsl1p and any other coatamer subunit. Thus, also in this study the direct interaction between Dsl1p and δ-COP was not shown.

In this application we have identified the binding sites in Dsl1p and δ-COP for one another and surprisingly found that Dsl1p and δ-COP, the polypeptide encoded by the yeast RET2 gene, bind each other directly and by a surprisingly strong and salt-stable interaction. Relatively short peptidic regions of Dsl1p and δ-COP bind to one another with high affinity and have proven useful as tags in affinity chromatography. These two peptides are therefore useful as tags for polypeptide purification and polypeptide detection and as tools for inducing the dimerization of two tagged polypeptides, even in vivo.

### SUMMARY OF THE INVENTION

The present invention relates to non-natural polypeptides selected from a pair of polypeptides which can bind to one another in the presence of high salt. In preferred embodiments these polypeptides can also be labeled with detectable groups.

The invention further relates to nucleic acids encoding these polypeptides.

The invention further relates to an affinity matrix comprising the non-natural polypeptides of the invention and a solid support. In preferred embodiments the polypeptides comprised by the affinity matrix are short, 10 to 50 amino acid long polypeptides. In another preferred embodiment the solid support of the affinity matrix is a commercially available solid support. Furthermore, the polypeptide can be coupled to an affinity matrix covalently or non-covalently.

The invention further relates to a kit comprising an affinity matrix of the invention and a container. In a preferred embodiment this kit further comprises an eluent.

The invention further relates to the use of a first polypeptide of the invention for the detection of a second polypeptide of the invention, whereby these two polypeptides are capable of binding to one another. In a preferred embodiment the polypeptide to be detected comprises a region with similarity to Dsl1p and is detected by a second polypeptide comprising a region with similarity to δ-COP or the other way round. In preferred embodiments the polypeptide used for detection is modified covalently with the detectable group.

The invention further relates to the use of a peptide derived from Dsl1p and a peptide derived from δ-COP for the dimerization of polypeptides. This means that a polypeptide comprising the sequence derived from Dsl1p will dimerize, even *in* vivo, with another polypeptide comprising a sequence derived from δ-COP. Induced dimerization in vivo by the interaction of the peptides with one another is a preferred embodiment of the invention. In another preferred embodiment this dimerization controls the biological activity of a polypeptide, for example if the first polypeptide changes its cellular localization after addition of the second polypeptide, which can be administered as a cell-permeable polypeptide, a polypeptide capable of being endocytosed, a polypeptide in a liposomal composition, or also by induced transcription of the second polypeptide. The biological activity of the first polypeptide of the invention can also be controlled, if the addition of the second polypeptide of the invention renders the first one non-functional. This can, e.g., occur if the addition of the second polypeptide leads to the degradation of the first polypeptide or its inhibition by any other means.

The invention further relates to a method for the purification of a polypeptide of the invention which comprises the steps of a) contacting a solution containing a polypeptide of the invention with an affinity matrix of the invention, whereby the affinity matrix comprises the other polypeptide of the pair of polypeptides according to the invention, b) washing the affinity matrix, and c) eluting the bound polypeptide. The wash of step b) can be a wash with an aqueous solution comprising salt and/or ionic and/or non-ionic detergent. Elution of the bound polypeptide can be done by an aqueous solution of glycine at low pH or with very high salt, e.g. 1 M NaCl. In a preferred embodiment the pH of the eluate is adjusted to an appropriate pH, e.g. between pH 4-9, 5-8 or 6-8 or any other pH appropriate.

### DETAILED DESCRIPTION OF THE INVENTION

In order that the invention described herein may be more completely understood, the following detailed description is provided. As used herein, the following definitions shall apply, unless otherwise indicated.

A "polypeptide" as used herein is a molecule comprising more than 10, in particular more than 15, 17, 20, 22, 25 or 27 and most preferably more than 30 amino acids, but less than 10000, in particular less than 9000, 8000, 7000, 6000, 5000, 4000, 3000 or 2000, most preferably less than 1500 amino acids. Also, polypeptides with substantial amino acid sequence identity and polypeptides, which contain a low percentage of modified or non-natural amino acids are encompassed.
A "non-natural" polypeptide is a polypeptide, which is not encoded as such by the genome of a naturally occuring species, in particular a polypeptide that is not identical to one of those polypeptides of the Genbank database as of September 3, 2002, with a naturally occuring species identified as their source. This means that a "non-natural" polypeptide does not occur as such in nature, but can be, and in particular was, produced by genetic engineering techniques. This term in particular encompasses all mutant polypeptides, in particular deletions, truncations, multiple substitutions and fusion polypeptides, which at one stage were produced by genetic engineering techniques. A "non-natural" polypeptide may comprise less than 200, in particular less than 190, 180, 170, 160, 150, 140, 130, 120, 110, 100, 90, 80, 70, 60, 50, 45 or 40, most preferably less than 35 amino acids of the wild-type Dsl1p protein sequence of *S. cerevisiae* or may comprise less than 200, in particular less than 190, 180, 170, 160, 150, 140, 130, 120, 110, 100, 90, 80, 70, 60, 50, 40, 35, 30, 25, 20 most preferably less than 15 amino acids of the wild-type δ-COP protein sequence of *S. cerevisiae.* The term "non-natural" polypeptide as used herein preferably does not include the fusion polypeptides GST-Dsl1p (aa1-754 and aa 1-724 fused to GST) as described in Andag et al., J. Biol. Chem. (2001) 42: 39150-39160 or fusion polypeptides of Dsl1p useful in so-called two-hybrid assays (e.g. aa 1-754 of Dsl1p fused to the Gal4p-binding domain as used in Reilly et al., Mol Biol Cell. (2001) 12:3783-96), or the fusion polypeptides of Ret2p described in Cosson et al., EMBO J. (1998): 17:6863-70 (e.g. aa 290-546, N-terminally fused with (His)6) or fusion polypeptides of Ret2p useful in so-called two-hybrid assays (e.g. as described in Cosson et al. above, e.g. aa 290-546 of Ret2p fused to the Gal4p-binding domain or as e.g. described by Reilly et al. above, e.g. aa 140-399, aa155-546 or aa120-546, each fused to the Gal4p-activation domain).

The term "at least X% identity with SEQ ID NO: 1 or SEQ ID NO:2" as used herein means that if a given polypeptide sequence is aligned to SEQ ID NO:1 or to SEQ ID NO:2, X% of the amino acids within the aligned region are identical. X can be at least 70, at least 75, at least 80, at least 85, at least 90 or at least 95. The closely related residues aspartic acid and glutamic acid are regarded as being identical, as well as the closely related residues asparagine and glutamine. However, preferably the closely related residues asparagine and glutamine are not regarded as being identical and more preferably the closely related residues aspartic acid and glutamic acid are also not regarded as being identical.

An "aqueous solution" as used herein is a solution comprising at least 70% water, particularly at least 80%, at least 85%, at least 90%, at least 95% or even at least 97% (all in %w/v).

The term "polypeptides capable of binding to one another" as used herein means that the two polypeptides of the invention bind to one another in such a way that the mobilized first polypeptide pulls down at least 10%, particularly at least 20%, at least 30%, at least 40%, at least 50%, at least 60%, at least 70%, at least 80% or even at least 90%, of the total amount of the second polypeptide. This can be tested, e.g., by immobilizing an excess of a purified GST-fusion-polypeptide of the first polypeptide, e.g. 500 pmol, on an appropriate amount on glutathione-Sepharose 4B, e.g. 20 µl bead solution, and then adding about 100 pmol of the purified second polypeptide in a total volume of 500 µl in preferably buffer A: 20 mM HEPES, 200 mM potassium acetate, 2 mM magnesium acetate, 1 mM DTT and 0,05 % Triton X-100, wherein the pH of buffer A is 6.8, or another suitable buffer, providing for essentially the same conditions with regard to pH, ionic strength and detergent concentration, and rotating for 2 hours at 4 °C. After four washes with 1 ml of, e.g., buffer A with a temperature of 4 °C - wherein the washes can be performed by adding the buffer, waiting for 2 min, gently centrifuging the sample, e.g. at 1000g for 3 min, and then removing the supernatant without removing the pelleted glutathione-Sepharose, all steps performed in a coldroom, preferably at around 4 °C. The total amount of the second polypeptide bound to the glutathione-Sepharose matrix can be determined, e.g. by boiling the beads in sample buffer and running the boiled sample on an SDS-gel side-by-side with a sample representing the input of the second polypeptide and comparing the bands representing the second polypeptide, after staining, e.g., with Coomassie Blue, e.g. by use of a scanner. Alternatively, the pair of polypeptides of the invention have a binding constant for one another lower than 5 µM, particularly lower than 1 µM, lower than 600 nM, lower than 300 nM, lower than 100 nM, or lower than 70 nM, most preferably from 0,1 nM to 50 nM. Binding constants can be determined, e.g., by standard methods like the BIACORE system according to the instructions of the manufacturer's manual in buffer A at 4 °C (Honing et al., J Biol Chem. (1997): 272(32):19884-90).

The term covalent "modification" refers to the incorporation of a detectable marker into the polypeptide of the invention, e.g. by attachment of a fluorophore, a chromophore, an enzyme, a radioisotope or the attachment of a moietiy that can be detected by a labeled second molecule containing a fluorophore, a chromophore, an enzyme or a radioisotope, in particular a fluorophore or an enzyme. Preferably the detectable marker or the enzymatic activity can be detected by an optical or colorimetric method. An example for such a two-step detection system is the well-known Biotin-Avidin system (Airenne et al., Biomol Eng. (1999) 16(1-4):87-92). Various methods suitable for the covalent modification of polypeptides are known in the art and may be used (for example see Lobl et al., (1988) Anal. Biochem., 170:502-511; Griffin et al., (1998) Science, 281(5374):269-72.

The term "affinity matrix" as used herein refers to any solid material usually used in chromatography to which a first polypeptide of the invention can be coupled, either covalently or non-covalently. The affinity matrix is in such a state capable of binding to the second polypeptide of the invention.

The term "dimerization" as used herein refers to the physical association of one polypeptide with at least one second polypeptide, preferably with only one second polypeptide.

The term "living cell" as used herein refers to bacterial, archaebacterial and eukaryotic cells, whether they are part of a living organism or are kept in cell culture. Under certain circumstances a "living cell" might not be part of a living human being.

The term "biological activity" refers to at least one of the activities that a polypeptide has or that it is known to control. For example, a polypeptide might have enzymatic activity, influence the enzymatic activity of another polypeptide by binding to it (and thereby either activate or inhibit the other polypeptide) or it may serve a structural function. One examples for a polypeptide with multiple activities is Aconitase, serving an enzymatic role in the citric acid cycle, but also a regulatory role in iron metabolism (Henderson and Kuhn, Prog Mol Subcell Biol. (1997) 18:117-39).

The term "cellular localization" as used herein refers to the steady-state localization of a polypeptide within a cell. For example, a polypeptide might be shuttling between the nucleus and the cytoplasm. If the majority of all polypeptides of this type of polypeptide at any given time is cytoplasmic, then the cellular localization of such a polypeptide would be regarded as being cytoplasmic. A change in cellular localization can occur, e.g., if, for one reason or the other, more polypeptides of this type of polypeptide were observed in the nucleus, leading to a change of the steady-state distribution of that polypeptide.

The term "non-functional" as used herein refers to the loss, or at least the reduction to less than 30% of the starting activity, of at least one of the biological activities of a polypeptide, preferably the loss of all biological activities of a polypeptide.

The present invention relates to a non-natural polypeptide selected from a pair of polypeptides, wherein one polypeptide comprises a sequence with at least 80% identity to SEQ ID NO:1 derived from Dsl1p, and another polypeptide comprises a sequence with at least 80% identity to SEQ ID NO:2 derived from the polypeptide δ-COP, wherein the two polypeptides are capable of binding to one another in an aqueous solution of 200 mM sodium acetate. The polypeptide comprising a sequence with at least 80% identity to SEQ ID NO:1 derived from Dsl1p is herein often also referred to as a D-type polypeptide. The polypeptide comprising a sequence with at least 80% identity to SEQ ID NO:2 derived from the polypeptide δ-COP, the polypeptide encoded by the yeast gene *RET2,* is herein often referred to as a R-type polypeptide. It has been found that these two types of polypeptides bind to one another very tightly, even in the presence of high salt, e.g. they still bind to one another in a solution with an ionic strength equal to 200 mM NaCl in H₂O, particularly equal to 250 mM NaCl in H₂O, 300 mM NaCl in H₂O, 350 mM NaCl in H₂O, 400 mM NaCl in H₂O, or even equal to 500 mM NaCl in H₂O. It is, of course, clear that the polypeptides of the invention also bind to one another in solutions with a lower ionic strength than 200 mM NaCl in H₂O, preferably when the ionic strength of the solution is higher than that of 0,1 mM NaCl in H₂O, more preferably higher than that of 1mM NaCl in H₂O, 5 mM NaCl in H₂O or 20 mM NaCl in H₂O. Ionic strength can be measured at pH 7,0 at a temperature of 25°C.

This strong interaction has been observed between polypeptides comprising the sequence SEQ ID NO: 1 and SEQ ID NO:2, but it is clear to a skilled person that starting from those two sequences many other sequences are derivable by the process of mutating a first polypeptide, then mutating a second polypeptide, and then selecting those second polypeptides which bind to the mutated first polypeptide. Such new functional pairs of D-type- and R-type-polypeptides are also encompassed by this invention, as long as they show at least 70%, particularly at least 75%, at least 80%, at least 85%, at least 90% or at least 95% amino acid identity with SEQ ID NO: 1 or SEQ ID NO:2, respectively, and as long as the new, derived polypeptides are still capable of binding to one another. The two polypeptides of a pair of polypeptides bind to one another at a pH between 4 and 9, preferably between 5 and 8,5, more preferably between 6 and 8, most preferably at a pH between 6,5 and 7,5.

In a preferred embodiment a non-natural polypeptide of the invention comprises a covalent modification, particularly wherein the covalent modification is a fluorophore, a chromophore, a radio label, an enzyme or a moiety that can be otherwise detected, e.g. by a labeled second molecule containing a fluorescent marker or enzymatic activity.

In a further embodiment the present invention relates to a nucleic acid encoding the non-natural polypeptide of the invention. The nucleic acid can, e.g., be part of a vector, e.g., a plasmid, a phagemid or a cosmid, more particularly a prokaryotic or eukaryotic expression vector (Sambrook et al., "Molecular Cloning: A Laboratory Manual", third edition, "Cold Spring Harbor Laboratory Press" (2001) for plasmids see 1.3-1.29; for phagemids see 3.42-3.52; for cosmids see 4.1-4.10; for mammalian expression vectors see 17.83-17.111). Such expression vectors comprise at least one promotor, at least one signal for translation initiation, at least one nucleic acid sequence of the invention and - in the case of prokaryotic expression vectors - a signal for translational termination; in the case of eukaryotic expression vectors such vectors preferably comprise additional signals for transcriptional termination and for polyadenylation.

Examples for prokaryotic expression vectors are, for expression in *Escherichia* coli, e.g. expression vectors based on promoters recognized by T7 RNA-polymerase, as described in US 4,952,496, for eukaryotic expression vectors for expression in *Saccharomyces cerevisiae,* e.g. the vectors p426Met25 or 526GAL1 (Mumberg et al. (1994) Nucl. Acids Res., 22, 5767-5768; Sikorski, R.S. et al. (1998) Genetics 122: 19-27), for the expression in insect cells, e.g. bacolovirus-vectors as e.g. described in EP-B1-0137839, and for the expression in mammalian cells, e.g., the vectors pRc/CMV or pRc/RSV (Gorman et al., Proc Natl Acad Sci U S A. (1982): Nov;79(22):6777-81; Invitrogen) or SV40-vectors (U.S. Pat. Nos. 5,292,658, 5,418,155) which are commonly known and commercially available. The molecular-biological methods for the production of these expression vectors, as well as the methods of transfecting host cells and culturing such transfected host cells as well as the conditions for producing and obtaining the polypeptides of the invention from those transformed host cells are well known to the skilled person (Sambrook, 3^{rd} edition (2001), chapter 16).

The present invention further relates to a host cell comprising a nucleic acid of the invention and/or a vector of the invention, particularly wherein the host cell is a microorganism like yeast or other fungi, like *Escherichia* coli, Bacillus *subtilis* or other bacteria. The host cell can also be a cell of higher eukaryotic origin, like a plant cell, an insect cell, preferably a virus-infected insect cell, more preferably a baculovirus-infected insect cell; or like a mammalian cell, like HeLa, COS, MDCK, 293, NS0, or a hybridoma cell.

The present invention further relates to an affinity matrix comprising the non-natural polypeptide of the invention and a solid support. Solid support in this respect is understood as any kind of solid material to which a polypeptide can be coupled, particularly those solid materials which are commonly used as a matrix in chromatography, like hydrophilic polyether resins, crosslinked styrene resins, crosslinked agarose resins, crosslinked cellulose resins, cellulose beads, granular cellulose, polystyrene resins, dextrane-based resins, agarose-based resins, G5000-based resins, hydrophilic gels, magnetic cellulose, cellulose-based resins, methacrylate-based resins, phenyl-ether-based resins, ethyl-ether-based resins, polymethacrylate-based resins, hydroxyapatite-based resins, resins based on silica particles, resins based on acrylic beads, and the like. The solid particles of the solid support have a size between 0,1 µm to 1 mm, preferably between 2 µm and 500 µm, more preferably between 5 µm and 200 µm. The solid particles of the affinity matrix should be stable in a pH range of between 6 and 8, more preferably between 5 and 9, even more preferably between 2 and 13.

In a preferred embodiment, the present invention relates to an affinity matrix comprising either a R-type-polypeptide, particularly a polypeptide which comprises SEQ ID NO:2, more particularly wherein the polypeptide comprises less than 30 amino acids, even more particularly less than 25, 20, 17 or 15 amino acids of δ-COP, most particularly wherein the polypeptide is SEQ ID NO:2; or a D-type-polypeptide, particularly a polypeptide which comprises SEQ ID NO:1, more particularly wherein the polypeptide comprises less than 50 amino acids, even more particularly less than 45, 40 amino acids, most particularly wherein the polypeptide is SEQ ID NO:1. The polypeptide comprised by the affinity matrix can also consist of less than 60, 55, 50, 45, or 40 amino acids, or, in the case of a polypeptide derived from δ-COP, even less than 35, 30, 25, 20 or 15 amino acids. The polypeptide comprised by the affinity matrix can consist of at least 10, 11, 12, 13, 14, 15, 16, 17, 18, 19 or 20 amino acids.

In a preferred embodiment, the solid support of the affinity matrix is selected from one of the solid materials described above and/or has a size as mentioned for the solid materials described above. The affinity matrix of the invention is useful for the purification of a first polypeptide of the invention, if the second of a pair of polypeptides is coupled to it, because one polypeptide of a pair of polypeptides of the invention shows affinity for the other polypeptide. A polypeptide of the invention can be coupled covalently or non-covalently to the solid support. An example for covalent coupling is the case in which the polypeptide of the invention is synthesized directly on the solid support by standard peptide chemistry but is never cleaved off. Covalent coupling can also occur between the lysines of a polypeptide of the invention and amino-functional groups of the solid support by, e.g., the use of certain bifunctional esters like dipimelidat esters, or it can be coupled to activated solid supports, like, e.g., NHS-activated Sepharose (van Sommeren et al. (1993) J Chromatogr. 639, 23). The polypeptide of the invention can also be coupled to the solid support via its cystein, aspartic acid and glutamic acid or serine residues. With regard to the chemistry of coupling a polypeptide to a solid phase, reference is made to Lundblad, R.L. (1991) Chemical reagents for protein modification, 2^{nd} edition, CRC Press; and Wong, S.S. (1991) Chemistry of protein conjugation and crosslinking. CRC Press, 248-251. A polypeptide can also be coupled to the solid support non-covalently, or indirectly. For example, the polypeptide of the invention can be modified with biotin and the modified polypeptide can then bind to a streptavidin-coated solid support, via the non-covalent interaction between biotin and streptavidin. In another example the polypeptide of the invention can also be modified with a peptidic tag, e.g., a FLAG-tag, and this modified polypeptide of the invention will then bind to a solid support coated with anti-FLAG-antibodies, via the non-covalent interaction between the FLAG-tag and the antibody.

The present invention further relates to a kit comprising an affinity matrix of the invention and a container. Such kits are often useful in polypeptide purification and then also comprise solutions useful for polypeptide purification, such as washing solutions and/or eluents. An eluent is a solution, preferably an aqueous solution, capable of breaking the interaction between a D-type polypeptide and an R-type polypeptide. Examples for such solutions are given below.

The present invention further relates to the use of an affinity matrix of the invention for the purification of a polypeptide of the invention. To do so, one polypeptide of a pair of polypeptides must be coupled to the affinity matrix and then the other of a pair of polypeptides can be purified with such an affinity matrix making use of the affinity of the two polypeptides for one another. This means that an affinity matrix with a coupled D-type polypeptide can bind to a polypeptide with a R-type polypeptide and be therefore used for purification of a R-type polypeptide, while an affinity matrix with a coupled R-type polypeptide can be used for the purification of a D-type polypeptide.
In a preferred embodiment, the polypeptide to be purified is expressed in a bacterium, a fungus or a higher eukaryotic cell, particularly in *Escherichia coli,* bacillus, e.g. Bacillus subtilis, yeast, e.g. *Saccharomyces cerevisiae,* plant cells, insect cells, mammalian cells, or the like. Any system suitable for expressing recombinant polypeptides can be used as starting point for purification. Expression of recombinant polypeptides in *E. coli* is described in Sambrook, 3^{rd} edition (2001), 15.1-15.60; expression of recombinant polypeptides in *B. subtilis* is described in Sambrook, 3^{rd} edition (2001), 15.55; expression of recombinant polypeptides in *S. cerevisiae* is described in Sambrook, 3^{rd} edition (2001), 18.1-18.108; expression of recombinant polypeptides in Plants is described in James E, Lee JM. (2001) Adv Biochem Eng Biotechnol. 72:127-56; expression of recombinant polypeptides based on Baculovirus is described in Sambrook, 3^{rd} edition (2001), 17.81-17.84; expression of recombinant polypeptides in Mammals is described in Sambrook, 3^{rd} edition (2001), chapter 17.

The high affinity of polypeptides of the D-type for polypeptides of the R-type makes it possible to use one type of polypeptides for the detection of the other type of polypeptides. Therefore, the present invention further relates to the use of a first polypeptide of the invention for the detection of a second polypeptide of the invention. In a preferred embodiment, the polypeptide to be detected is a D-type-polypeptide, and the polypeptide to be used for detection is a R-type-polypeptide, particularly SEQ ID NO:2; or the polypeptide to be detected is a R-type-polypeptide, particularly SEQ ID NO:2, and the polypeptide to be used for detection is a D-type-polypeptide, particularly SEQ ID NO:1. That is to say that a D-type polypeptide can be detected by a R-type polypeptide, and vice versa. In a preferred embodiment, the polypeptide used for detection is a modified polypeptide of the invention. This means that if a D-type polypeptide is to be detected, then preferably a modified R-type polypeptide, e.g. a fluorescently or otherwise labeled R-type polypeptide should be used for detection, and vice versa.

The high affinity of D-type polypeptides for R-type polypeptides can be used to dimerize D-type polypeptides with R-type polypeptides *in vivo.* The present invention therefore further relates to the use of a first polypeptide of the invention for the dimerization with a second polypeptide of the invention. In particular, the first polypeptide can be a D-type-polypeptide, particularly SEQ ID NO:1, and the second polypeptide is then a R-type-polypeptide, particularly SEQ ID NO:2; or the first polypeptide can be a R-type-polypeptide, particularly SEQ ID NO:2, and the second polypeptide is then a D-type-polypeptide, particularly SEQ ID NO:1.

In a preferred embodiment, dimerization of the D-type polypeptide with the R-type polypeptide occurs within a living cell.

The dimerization between the D-type-polypeptide and the R-type-polypeptide within a living cell can be used for detecting one or the other type of polypeptides in a living cell, e.g. by using a GFP-tagged D-type-polypeptide to detect an unlabeled R-type-polypeptide, and vice versa. This might be useful where it is detrimental to use a GFP-tagged version of the polypeptide to be detected directly, e.g. where such a GFP-tagged version would be non-functional. In such a scenario, a small amount of the GFP-tagged D-type-polypeptide would be sufficient to indirectly monitor the functional R-type-polypeptide, while minimally interfering with the function of the R-type-polypeptide, and vice versa.

In one preferred embodiment, however, the dimerization between D-type-polypeptides and R-type-polypeptides controls the biological activity of either D-type-polypeptide or R-type-polypeptide, or both. That is to say, one particular function of D-type-polypeptide could be activated or inhibited or in other ways regulated upon dimerization with the R-type-polypeptide, and vice versa. For example, the D-type-polypeptide could be an enzyme, which can be inhibited upon dimerization with an inhibiting R-type-polypeptide or activated upon dimerization with a R-type cofactor. An example for such a polypeptide regulated by dimerization is the enzyme bacterial phospholipase A, an integral membrane phospholipase: its activity is strictly regulated by reversible dimerisation (Snijder et al. (2000) Biochim Biophys Acta 1488(1-2):91-101). An example for inhibition by a second polypeptide is the Inhibition of Rho family GTPases by Rho GDP dissociation inhibitor (Wei et al., (2002) Development. 129(7):1705-14.

In one preferred embodiment, the first polypeptide changes its cellular localization after addition of the second polypeptide. This is to say, for example, a D-type-polypeptide might be nuclear at steady state. Now if a shuttling R-type-polypeptide with a functional nuclear export signal is added to the living cell, the D-type polypeptide could change its localization, e.g., from nuclear to cytoplasmic. This change of localization could lead to functional inactivation of the first polypeptide, e.g., if the first polypeptide is a transcription factor, it is functionally inactivated if moved out of the nucleoplasm, e.g., to the cytoplasm after dimerization with the second polypeptide. Of course, a change of localization could also lead to activation of the first polypeptide, e.g., if the first polypeptide is a D-type polypeptide, e.g. a transcription factor with a non-functional nuclear localization signal (NLS), then the addition of a second, R-type polypeptide with a functional NLS could lead to nuclear localization of that transcription factor, and thereby to the activation of the first polypeptide. For review of different types of nuclear localization signals and nuclear export signals see Mattaj and Englmeier (1998) Annu Rev Biochem. 67:265-306 and Görlich and Kutay (1998) Annu Rev Cell Dev Biol. 15:607-60. Of course, the first polypeptide can also be an R-type polypeptide, then the second polypeptide is a D-type polypeptide. Of course, changes of polypeptides in other cellular localizations are also encompassed by the invention. For example C-terminal tagging of an otherwise secreted protein with an ER-retrieval signal leads this protein from the early Golgi compartment back to the ER. Thus, its secretion is inhibited with high efficiency (Letourneur et al., Cell. (1994): 79(7):1199-207). In turn, tagging an ER-resident protein with an ER-export signal forces this protein to leave the ER (Votsmeier and Gallwitz, EMBO J. (2001): 20(23):6742-50).As another example, the first polypeptide can, e.g., be a surface receptor, which will be down-regulated by the addition of the second polypeptide which bears a signal for endocytosis, or a first , initially cytoplasmic, polypeptide will be moved to the membrane and into proximity of signaling complexes by the addition of a second polypeptide bearing a signal for membrane attachment. It is preferred that the first polypeptide is initially present in the cells without the second polypeptide and then, if one desires to do so, the second polypeptide is administered to the cell. The second polypeptide can be administered as a cell-permeable polypeptide, a polypeptide capable of being endocytosed, a polypeptide in a liposomal composition, or a polypeptide synthesized de novo within the cell by induced transcription. Cell-permeable polypeptides are known from Hawiger J. (1999) Curr Opin Chem Biol 3(1):89-94. Their utility is based on the observation that polypeptides, which bear certain peptidic signals can translocate through the cytoplasmic membrane into the cytoplasm. Thus, the first polypeptide can be induced at any given time to dimerize with the second polypeptide, if the second polypeptide is administered as a cell-permeable polypeptide, e.g. as a purified recombinant cell-permeable polypeptide to the cell culture in which the first polypeptide is expressed. The second polypeptide can also be administered as a purified recombinant polypeptide to the cell culture in which the first polypeptide is expressed, if it carries signals for endocytosis, as described in Kirchhausen T. (2002) Cell 109(4):413-6 and is therefore capable of being endocytosed. The second polypeptide can also be delivered to the interior of a cell as a polypeptide enclosed in a liposomal composition, that is, the recombinant second polypeptide can be trapped, e.g. by sonication, in the inside of lipid vesicles, which then fuse with the cell membrane and deliver the second polypeptide to the interior of a cell in culture. Methods for preparing liposomes are well known in the art and e.g. described in Chatterjee and Banerjee (2002) Methods Mol Biol. 199:3-16. Of course, the second polypeptide can also be administered to the interior of a cell by inducing the expression of a regulated version of the gene for the second polypeptide. This can be achieved, e.g. in the case of mammalian cells, by cotransfecting a construct for a second polypeptide together with a construct for the first polypeptide, wherein the gene encoding the second polypeptide is under the control of an inducible promoter. Examples for inducible promoters are, e.g., the lac-promoter in *E. coli* (Skelly and Madden (1996) Methods Mol Biol. 56:23-53, the GAL10-promoter in *S. cerevisiae* (Mitchell et al. (1993) Yeast 9(7):715-22. Inducible promotors in insect cells are described by Wu et al. (2000) J Biotechnol. 80(1):75-83 and inducible promotors in mammalian cells are described by Yarranton (1992) Curr Opin Biotechnol. 3(5):506-11.

In a preferred embodiment, the first polypeptide is rendered non-functional by the addition of the second polypeptide, particularly if the first polypeptide is degraded or inhibited after addition of the second polypeptide. Degradation of the first polypeptide can be, for example, achieved if the second polypeptide carries signals for its own destruction, e.g. signals for polyubiquitination, and thereby drags the first polypeptide to the proteasome for destruction, or if the second polypeptide carries signals leading to the polyubiquitination of the first polypeptide, thus leading directly to the destruction of the first polypeptide. The second polypeptide can also inhibit the first polypeptide in other ways, for example, if binding of the second polypeptide sterically interferes with substrate binding of the first polypeptide or, as described above, with the localization needed for proper activity of the first polypeptide. The second polypeptide can also be modified with agents, like malachite green, which upon illumination with the proper wavelength produce oxygen radicals leading to the inactivation of a polypeptide in close proximity (Jay DG (1988) Proc Natl Acad Sci U S A. 85(15):5454-8). Thus, if a second polypeptide labeled with malachite green is dimerized with the first polypeptide then irradiation of the cells with the proper wavelength will lead to functional inactivation of the first polypeptide as well.

The invention further relates to a method for the purification of a polypeptide of the invention comprising the following steps: a) contacting a solution containing a polypeptide of the invention with an affinity matrix of the invention, wherein the polypeptide of the affinity matrix is the other polypeptide of the pair of polypeptides according to the invention, that is, if the polypeptide to be purified is a D-type polypeptide, then the polypeptide of the affinity matrix must be an R-type polypeptide, and vice versa, b) washing the affinity matrix, and c) eluting the bound polypeptide.

The solution containing the polypeptide to be purified can be a crude lysate of a culture of host cells in which the polypeptide of the invention has been expressed to detectable levels, or it can also be a solution, which has already undergone one or more purification steps, like centrifugation of debris, ion-exchange chromatography, metal-affinity chromatography, or the like. Such a solution can be brought into contact with the affinity matrix, for example by loading the solution onto a chromatographic column packed with the affinity matrix of the invention, or by adding the affinity matrix of the invention to a solution to be purified, in order to allow binding of the polypeptide to be purified in a so-called batch-mode. Preferably, the time of contact between the solution and the affinity matrix is long enough to allow binding of the polypeptide to be purified to the affinity matrix. This means for the purification scheme in column-mode that the solution to be purified is loaded onto the column with a flow-rate of less than 5 column volumes per minute, particularly less than 2 column volumes per minute, more particularly less than 0,5 column volumes per minute, even more particularly less than 0,1 column volumes per minute. If purification is in batch-mode then the time allowed for contact between the solution and the affinity matrix can be more than 5 minutes, but less than 10 days, particularly more than 10 minutes, but preferably less than 2 days, more particularly at least 20 minutes. The solution containing the polypeptide to be purified should have an ionic strength equaling to less than 700 mM NaCl in H₂O, particularly less than 500 mM NaCl in H₂O, 400 mM NaCl in H₂O, 350 mM NaCl in H₂O, 300 mM NaCl in H₂O, 250 mM NaCl in H₂O or 200 mM NaCl in H₂O. It is, of course, clear that the polypeptides of the invention also bind to one another in solutions with a lower ionic strength than 200 mM NaCl in H₂O. Therefore, the ionic strength of the solution should preferably be higher than the ionic strength of a solution of 0,1 mM NaCl in H₂O, more preferably higher than that of 1mM NaCl in H₂O, 5 mM NaCl in H₂O or 20 mM NaCl in H₂O. Binding should occur at a suitable temperature, particularly at low temperatures, but above the freezing point, more particularly between 0,1 °C and 25 °C, more particularly between 0,1 °C and 15 °C, even more particularly between 0,1 °C and 10 °C. The solution preferably contains protease inhibitors, like, e.g., aprotinin, pepstatin, leupeptin, DMSF and the like, e.g. as described in Current Protocols in Molecular Biology, 1996; 13.13.7 and 13.13.8. Protease inhibitors and the concentrations at which they should be applied are well known in the art. The solution to be purified should have an appropriate pH, preferably between 3,5 and 10, more preferably between 5 and 9, even more preferably between 6 and 8. The column, or the loose affinity matrix in the case of batch-mode purification, can be washed with aqueous solutions with an ionic strength higher than that of 10 mM NaCl in H₂O, 50 mM NaCl, 150 mM NaCl, 250 mM NaCl, 350 mM NaCl, 500 mM NaCl, or up to 600 mM NaCl in H₂O, as long as the ionic strength of the washing solution is lower than that of 750 mM NaCl in H₂O, preferably lower than that of 700 mM NaCl in H₂O, more preferably lower than that of 650 mM NaCl in H₂O. The aqueous solution for the wash can comprise at least 50 mM NaCl, 150 mM NaCl, 250 mM NaCl or even 500 mM NaCl in H₂O. The solution for the wash can be buffered, e.g. by Tris, HEPES, phosphate-based buffers, MOPS or other buffer systems known in the art, e.g. described in Current Protocols in Molecular Biology, 1996; A.2.1-A.2.6. The pH of the solution for the wash can be between 4 and 10, particularly between 5 and 9, more particularly between 6 and 8. Of course, other anions than chloride can be used, e.g. like acetate, phosphate, hydrogen phosphate, carbonate, hydrogen carbonate, lactate or others. Of course, also other cations than sodium can be used, like lithium, potassium, magnesium, calcium or other metal ions, particularly alkaline or earth alkaline metal ions, but also non-metal cations like ammonia or other nitrogen-based cations. The solution for the wash of step b) may also comprise detergents, e.g. non-ionic, anionic, zwitterionic or amphoteric detergents. Particularly less than 2% detergent, more particularly less than 1% detergent, even more particularly less than 0,5% detergent can be comprised, but also more than 0,05% detergent, even more particularly more than 0,2% detergent or even around 1% detergent, particularly 1% non-ionic detergent may be used as detergent. Particularly preferred detergents are Triton X-100, Tween, Nonidet P40 and others, e.g. as described in Current Protocols in Molecular Biology, 1996; 10.15.11 and 10.15.12.

The polypeptide bound to the second polypeptide of the affinity matrix can be eluted by all substances that break the polypeptide-polypeptide interaction. Elution can be by denaturing agent, like SDS, urea or guanidinium hydrochloride, used at concentrations sufficient for the denaturation of proteins, but in preferred embodiments elution is by aqueous solutions which keep the eluted polypeptide functional. This can be achieved by aqueous solutions comprising 100 mM glycine or 1 M sodium chloride. Of course, also solutions comprising 1,5 M NaCl, 2 M NaCl or even more elute the bound polypeptide, but it is preferable to use the most gentle conditions sufficient for elution. This means that elution is preferred by buffered aqueous solutions with an ionic strength from 0,8 M to 2 M NaCl, more preferably from 0,9 M to 1,5 M NaCl. Another preferred method for elution is elution with a polypeptide of the affinity matrix, that is a D-type polypeptide bound to a R-type affinity matrix can be eluted with an R-type peptide, and vice versa. The concentration of such a peptide for elution should be between 1 µM and 50 mM, preferably between 10 µM and 5 mM, more preferably between 50 µM and 2 mM. Also, tryptophane can be used for gentle elution of the bound polypeptide. Preferred concentrations of tryptophane are between 50 µM and 500 mM, more preferably between 1 mM and 400 mM, even more preferably above 10, 20, 30, 40, 50, 60, 70, 80, 90 or 100 mM tryptophane.

Elution can also be with a solution comprising from 20 mM to 2M glycine, particularly between 50 mM to 1M glycine, more particularly between 60mM to 500 mM glycine, even more particularly around 100 mM glycine, wherein said solution can have a pH below pH 3,5, particularly a pH from 1,0 to 3,0, more particularly from 2,0 to 3,0, most particularly around pH 2,5. In such a case, the pH of the eluate is then adjusted to between pH 4-9, more preferably pH 5-8, even more preferably pH 6-8 after elution, by the addition of, e.g. an appropriate amount of Tris-base or another base. This is preferred to keep the eluted polypeptide in a functional state.

If elution was by glycine, a polypeptide, tryptophane or high salt, the eluted polypeptide can be transferred into a buffer suitable for storage by the use of standard techniques, like dialysis against a suitable storage buffer or gelfiltration on a column pre-equilibrated with a suitable storage buffer; also microcentrifuge devices for buffer exchange can be used. Of course, after purification of the polypeptide of the invention on an affinity matrix of the invention, further purification steps can be performed, if appropriate. By way of example, the eluate of step c) can be used as the starting material for ion-exchange chromatography, gel filtration, hydrophobic interaction chromatography, or the like.

### DESCRIPTION OF THE DRAWINGS

The following examples, including the experiments conducted and results achieved, are provided for illustrative purposes only and are not to be construed as limiting for the present invention.

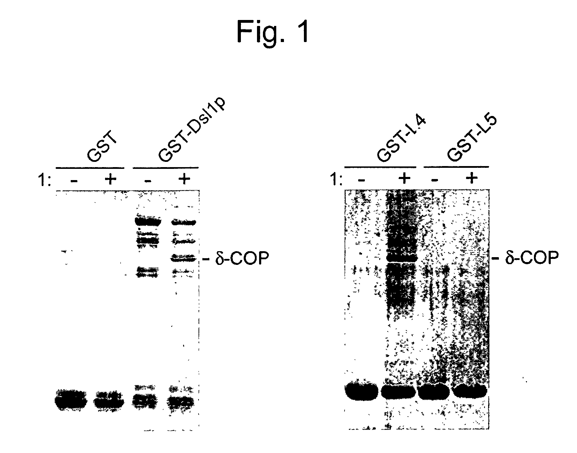

### Fig. 1. Specific binding of Ret2p (= δ-COP) to GST-Dsl1p and GST-L4.

Proteins from detergent-lysed yeast cells overexpressing *RET2* (1) were incubated at 4°C for 2 h with GST alone, GST-Dsl1p and GST-L4 (L4 = AVSKD DDWNWEVEDD DADAWGDEID VNIDDEEEKT; "WxW"; SED ID NO: 1) or GST-L5 (L5 = AVSKD DDANWEVEDD DADAWGDEID VNIDDEEEKT; "AxW") purified from *E. coli* and immobilized on glutathione-Sepharose 4B. Beads were washed 5 times, and the proteins retained were analyzed by SDS-PAGE followed by Coomassie Blue staining. Lanes marked with "-" represent incubations with buffer, whereas lanes marked with "+" represent incubations with extract of *RET2*-overexpressing cells. The position of the δ-COP subunit is indicated.

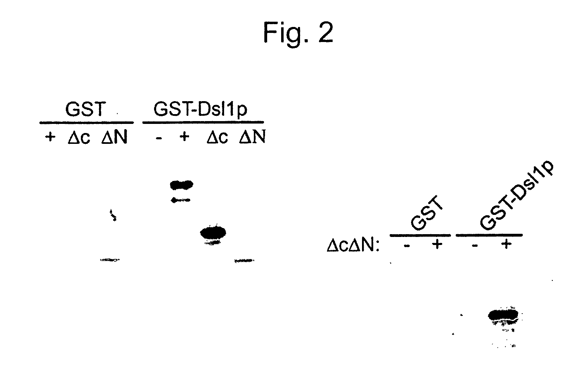

### Fig. 2. Ret2p/δ-COP interacts with Dsl1p via a central region of low structural complexity.

GST/GST-Dsl1p were loaded on glutathione-Sepharose 4B and incubated with buffer (-) or with either full length (+) recombinant His₆-tagged δ-COP or C-terminal (Δc; aa 2-400), N-terminal (ΔN; aa 281-546) as well as C- and N-terminal (ΔcΔN; aa 190-400) truncations of His₆-δCOP. Samples were washed 5 times and analyzed for bound δ-COP and δ-COP fragments by immunoblot analysis using specific antibodies raised against Ret2p/δ-COP.

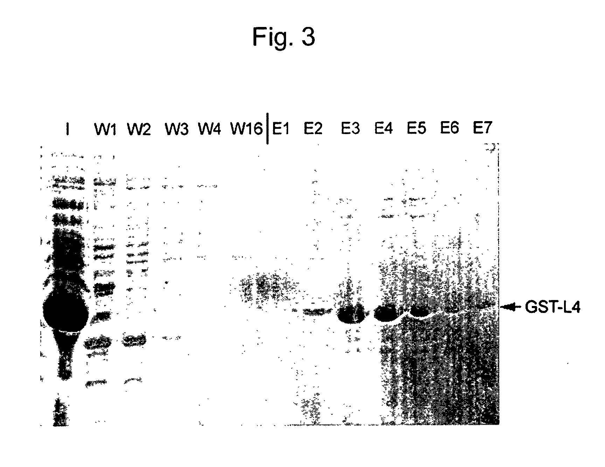

### Fig. 3. Specific binding of a Ret2p-derived peptide to the "WxW" motif of Dsl1p.

Peptides spanning the region from arginine 242 to glycine 254 of δ-COP (= SED ID NO 2) were immobilized on agarose and incubated at room temperature with crude extract of GST-L4 (L4 = SED ID NO 1) expressing (see Fig. 2) *E. coli* cells. After washing thoroughly bound proteins were eluted (for details see "Materials and methods"). An aliquot of the total input (I) and fractions of washing (W) as well as elution (E) steps were collected and analyzed by SDS-PAGE followed by Coomassie Blue staining. The position of the GST-L4 protein is indicated.

### EXAMPLES

### Example 1: Dsl1p binds δ-COP in vitro

A GST-Dsl1p fusion construct expressed in *E. coli* was immobilized on glutathione-Sepharose beads and incubated with extracts from wild type yeast cells. To determine, which of the different coatomer subunits interacts directly with Dsl1p we used extracts from yeast cells overproducing single subunits of the COPI coat. The binding efficiency of coatomer to GST-tagged Dsl1p was not improved by using extracts of cells overexpressing either α- or β'-COP (data not shown). In contrast, overexpression of the δ-COP-encoding *RET2* gene increased the amount of δ-COP retained on GST-Dsl1p beads dramatically (Figure 1, left panel). The protein was detectable by Coomassie staining. Immunoblot analysis was performed to confirm that this band in fact represents δ-COP (data not shown). α-COP binding was elevated when δ-COP was overproduced (Figure 1, left panel). This result indicates that δ-COP mediates the coatomer-Ds11p interaction.

### Example 2: Dsl1p binds to δ-COP via its central acidic domain

Several Dsl1p fragments were fused to GST and analyzed for their ability to bind δ-COP. The C-terminal part of Dsl1p (aa 461-754) was not able to bind significant amounts of δ-COP. In contrast, the N-terminal portion (aa 1-445) bound δ-COP very efficiently. Next we analyzed three overlapping fragments derived from the N-terminal part of Dsl1p to localize the binding region more precisely. Only the fragment spanning residues 301 to 449 of Dsl1p bound to δ-COP (data not shown). This region harbors a di-aromatic sequence motif with only one asparagine residue between the two tryptophans (-D-D-D-W-N-W-E- = "WxW"). To test the functional significance of this "WxW" motif, we fused this aspartic acid-rich domain (aa 406-440) to GST and analyzed this chimeric protein (GST-L4; L4 = SED ID NO 1) for interaction with δ-COP. A single W→A substitution of tryptophan 413 ("AxW"; L5) completely abolished the interaction of this Ds11p fragment to δ-COP (Figure 1, right panel).

Since a second tryptophan-containing region ("W⁴⁵⁵xxxW⁴⁵⁹") in close proximity to the "WxW"-motif(SED ID NO 1) contributes to the interaction with δ-COP, W→A substitutions in the "WxxxW"-region were also used to monitor for potential effects. To access the relative importance of the "WxW" and "WxxxW" motifs, single and double W→A substitutions (*dsl*1^{W413A}; *dsl1*^{W455A}; *dsl1*^{W413A/W455A}) were tested for their ability to reduce interaction with coatomer when introduced into full length GST-Dsl1p. The W455A single substitution ("AxxxW") did not significantly reduce the extent of binding to δ-COP. In contrast, the *dsl1*^{W413A} mutant protein ("AxW") showed a strongly reduced binding compared to wild type. The double mutant ("AxW...AxxxW") had the strongest effect and showed only negligible δ-COP binding in *vitro* (data not shown).

We confirmed these results by yeast two-hybrid analysis (Fields and Song; Nature. (1989) 340(6230):245-6). The C-terminal part of the δ-COP-encoding *RET2* gene (starting at codon 156 residue, isolate 2, Reilly et al; Mol Biol Cell. (2001) 12:3783-96) fused to the sequence of the Gal4p activation domain was used as prey. The different *DSL1* versions, wild type or W→A substitutions, fused to the Gal4p-DNA binding domain served as bait. In this assay the formation of a complex between the GAL4 activation and GAL4 DNA binding domain confers prototrophy for adenine. The expression of the W455A substitution ("AxxxW") resulted in growth similar to wild type, whereas the W413A ("AxW") substitution led to a strongly decreased interaction and therefore much slower growth (data not shown). Consistent with data from pull-down experiments, substitution of both tryptophan residues (W413A + W455A = "AxW...AxxxW") reduced the growth rate to that of the vector control (data not shown).

### Example 3: In vivo effects of alanine substitutions at W413 and W455 in Dsl1p

We wanted to determine whether the strong effect of the W→A substitution observed in vitro is mirrored by phenotypic changes in vivo. Therefore, we performed a plasmid shuffling experiment. The mutations described above were introduced into the *DSL1* sequence present on a low copy plasmid (*LEU2* marker). The plasmids obtained were transformed into a *Δdsl1* cell expressing a wild type *DSL1* from another vector carrying the *URA3* marker. Cells harboring the latter plasmid are Ura⁺ and thus sensitive to the drug 5-FOA (5-fluoroorotic acid; Boeke et al.; Mol Gen Genet. (1984) 197(2):345-6). After replica plating onto 5-FOA containing plates cells were analyzed for growth. Only those cells, which were able to loose the *URA3* + *DSL1* containing plasmid, can survive. Both single mutants, *dsl1*^{W413A} and *dsl1* ^{W455A}, ("AxW" or "AxxxW") showed growth on 5-FOA plates suggesting that they can replace the wild type *DSL1* gene very efficiently (data not shown). In contrast, the *dsl1*^{W413/AW455A} expressing cells ("AxW...AxxxW") were not able to form colonies on these plates. The apparent lack of function of the double mutation was confirmed independently: *Δdsl1* spores *(dsl1::kanMX)* expressing this mutant allele from a centromeric vector could not form colonies. The same plasmid was also not able to suppress the *ts*⁻-phenotype of *dsl1-22* cells (Andag et al.; J Biol Chem. (2001) 276(42):39150-60; data not shown).

The single W→A substitutions (W413A; W455A) did not lead to visible growth defects in a temperature range from 14° C to 37° C (data not shown). We examined possible additive effects by combining the single mutations in *DSL1* with the *ret2-1* (δ-COP) mutation. Double mutants *dsl1*^{*W413A*}*ret2-1* and *dsl1*^{W455A}*ret2-1* grew very slowly at 25°C and were unable to form colonies at 33°C, a permissive temperature for the single mutants (data not shown). No synthetic interaction was observed when we combined the same *dsl1* mutations with a mutation bearing a defect in the γ-COP subunit of coatomer, *sec21-1* (Hosobuchi et al.; Nature. (1992) 360(6404):603-5). This indicates that the synthetic growth defect observed with *dsl1*^{W413A}*ret2-1* and *dsl1*^{*W455A*}*ret2-1* mutants is specific. In contrast, the *dsl1-22* mutation showed synthetic interaction with both the ret2-1 and the *sec21-1* mutation (Andag et al., 2001). Together these results indicated that the single *dsl1*^{W413A} and *dsl1*^{W455A} mutants are not fully functional.

A hallmark of mutants affected in the Golgi-ER retrograde traffic mutants is the secretion of the soluble ER protein BiP/Kar2p (Semenza et al.; Cell. (1990) 61(7):1349-57). This is also true for *dsl1-22*, *dsl1-4* and *dsl1-7* mutants (Andag et al., 2001; Reilly et al., 2001). We examined BiP/Kar2p secretion by wild type cells *(DSL1)* or *dsl1-22,* dsl1^{W413A} and *dsl1*^{*W455A*} mutants. Cells were grown at 33°C on nitrocellulose filters that were placed on top of rich media plates for 18 h. Subsequent immunodetection revealed that the extent of BiP/Kar2p of *dsl1*^{W413A} ("AxW") cells was comparable to that of *dsl1-22* cells (Andag et al., 2001). The *dsl1*^{W455A} ("AxxxW") mutation did not increase BiP/Kar2p secretion above wild type level (data not shown). This finding suggests that the decrease in δ-COP binding of *dsl1* mutants observed in vitro correlates well with the in *vivo* effects on growth and retrograde transport from Golgi to ER.

In summary, tryptophan residues W413 and W455 within the central acidic region of Ds11p are important for the interaction with δ-COP and for viability. Alanine substitution of tryptophan 413 ("AxW") has stronger effects in vivo and *in* vitro than the W455A ("AxxxW") mutation.

### Example 4: Dsl1p binds to a central region within the δ-COP

GST-Dsl1p produced in *E. coli* was immobilized on glutathione-Sepharose beads for binding experiments. The following His₆-tagged versions of δ-COP produced in *E.* coli and applied to the in GST-Ds11p carrying beads: full length δ-COP, a N-terminal truncation (ΔN, aa 281-546), a C-terminal truncation (Δc, aa 2-400) and a segment derived from the center of δ-COP (ΔcΔN, aa 190-400). As shown in Figure 2, full-length δ-COP, δ-COP-Δc (aa 2-400) and ΔcΔN (190-400) were able to bind to Dsl1p. No binding of δ-COP-ΔN (aa 281-546) to GST-Dsl1p could be observed (Figure 2). This suggested that the central part of δ-COP spanning about 100 residues around residue 240 might be required for the binding to Dsl1p.

To further delineate the Dsl1p binding domain in δ-COP 13-mer peptides spanning amino acids 191-302 of δ-COP were synthesized on a cellulose membrane (overlap in sequence by 10 amino acids; Jerini; Berlin, Germany) and probed with to GST-Dsl1p. Anti-Dsllp antibodies were used to detect bound GST-Dsl1p. A peptide representing residues 242 to 254 of δ-COP (R-type-peptide; SED ID NO 2 (RAGHSATGGMKLG)) gave the strongest signal (data not shown). We cannot exclude that peptides from position 248 to 270 also bind to Dsl1p since anti-Dsl1 antibody alone gave already a weak signal with peptides derived from this region. To verify these data, peptides spanning the region from arginine 242 to glycine 254 of δ-COP (R-peptide; SED ID NO 2) were synthesized and immobilized on agarose. To this resin we applied crude extract of *E. coli* cells producing a chimeric protein consisting of GST and the δ-COP binding domain (GST-L4, "WxW"; L4 = D-type-polypeptide; SED ID NO 1 (AVSKDDDWNWEVEDDDADAWGDEIDV-NIDDEEEKT)). After washing thoroughly, bound proteins were eluted and analyzed by SDS-PAGE followed by Coomassie staining. The result obtained with crude extract of *E. coli* cells producing GST-L4 clearly illustrates the high specificity of the interaction (Figure 3). GST-L4 (L4 = D-peptide; SED ID NO 1) was retained very efficiently by the δ-COP peptide (R-peptide; SED ID NO 2), while most of the endogenous *E. coli* proteins were removed from the column by the washing steps.

### MATERIALS AND METHODS

### Bacterial and yeast strains

Cloning experiments were performed with the *E. coli* strain DH5α. Expression of either (His)₆-tagged or GST fusion proteins was performed using protease-deficient strains BL21DE3 or C41DE3 as described previously (Andag et al., 2001). *Saccharomyces cerevisiae* strains used are listed in Table I. Cells were grown in yeast extract-peptone-dextrose or synthetic minimal medium containing glucose (2%) as carbon sources and supplemented as necessary with 20 mg/l tryptophan, histidine, adenine, uracil, or 30 mg/l leucine or lysine. 5-FOA plates were prepared as synthetic minimal medium containing 0.1 % 5-FOA. Yeast transformations were performed as described previously (Elble; Biotechniques. (1992) 13(1):18-20). Standard techniques were used for complementation analysis, sporulation and the analysis of tetrads (Current Protocols in Molecular Biology, 1996; Vol.2; chapter/page: 13.2.1 - 13.2.9).

### Cloning and Plasmids

Plasmids used in this work are listed in Table II. Recombinant wild type and mutant Ds11p/Ds11p-fragments were expressed as GST- (glutathione S-transferase) fusion proteins in pGEX-TT (pGEX-2T with modifications in the polylinker region; Amersham Pharmacia Biotech). *DSL1* and the different truncations were amplified using pUA73 (*DSL1*-pRS315) as template and *Pfu* DNA polymerase (Stratagene) by polymerase chain reaction (PCR). Primer with a flanking *Xma*I site (5') and *Xba*I site (3') were used to generate the desired truncations for integration into the pre-digested vector pGEX-TT. Alanine substitutions were generated by site-directed mutagenesis using the QuikChange kit (Stratagene). *DSL1*-fragments L1-L5 were generated by annealing of oligonucleotides with flanking *Bam*HI (5') and *Hind*III sites (3') and integrated into the pre-digested vector pGEX-TT.

Full length Ret2p/δ-COP and its truncations were expressed as N-terminal (His)₆-fusions from pQE30 plasmid (QIAGEN). The primers used for the PCR created *Bam*HI (5') and *Hind*III (3') sites flanking the PCR product. These sites were used for its integration into the pre-digested vector.

### Purification of recombinant proteins and affinity-binding assay

*E. coli* cells expressing GST fusion proteins were lysed and proteins were solubilized in lysis buffer (20 mM HEPES, pH 6.8, 150 mM KOAc, 5 mM Mg(OAc)₂, 1 mM DTT, 1 % Triton X-100, protease inhibitor mix). GST fusion proteins were immobilized on glutathione-Sepharose 4B and washed 5 times with 10 volumes lysis buffer. E. coli strains expressing (His)₆-fusion proteins were lysed and proteins were solubilized in phosphate buffer (50 mM Na₂HPO₄, pH 8.0, 300 mM NaCl, 0.5 % Triton X-100, protease inhibitor mix). (His)6-tagged proteins were immobilized on Ni-NTA agarose, washed 5 times with 10 volumes phosphate buffer and eluted with 250 mM imidazol.

*E. coli* proteins immobilized on glutathione-Sepharose 4B were incubated at 4 °C for 2 h either with 100,000 x g supernatant of indicated yeast cell lysate or purified recombinant (His)6 tagged proteins. The beads were washed 5 times (10x beads volume each washing step) and proteins were separated by SDS-PAGE followed by Coomassie-/silver staining (Merril et al., (1983) Methods Enzymol. 96: 230-239) or immunoblot analysis using the ECL detection system (AmershamPharmaciaBiotech).

### Peptide spot overlay assay/peptide binding experiments

To analyze the Ds11p binding region within the Ret2 protein overlapping 13-mer peptides (overlap 10 residues each) spanning the region between residue 191 and 302 were directly synthesized on a cellulose membrane (Jerini; Berlin, Germany). The membrane was incubated in blocking reagent (1% BSA in Tris/HCl-buffer: 10 mM Tris/HCl, 0.9% (w/v) NaCl, 0.5% (v/v) Triton X-100) overnight at room temperature, followed by three washes with Tris/HCl-buffer. After 2 h incubation with purified GST-Ds11p in blocking buffer, the membrane was washed three times with Tris/HCl-buffer and bound GST-Ds11p was detected by using α-Dsl1p antibodies and the ECL detection system (AmershamPharmaciaBiotech).

14- to 27-mer peptides containing a N-terminal cysteine residue for the later coupling step were synthesized by the Fmoc (N-(9-fluorenyl)methoxycarbonyl) method on a Biosearch 9050 Pep-synthesizer (Milligen). Removal of protecting groups and cleavage of peptides from the resin was achieved by treatment with a solution of 90% trifluoroacetic acid, 5% water, 2.5% phenol, and 2.5% 1,2-ethanedithiol (all from Sigma). Peptides were immobilized on agarose gel using the SulfoLink kit (Pierce) following the instructions of the manufacturer. Immobilized peptides were incubated at room temperature with indicated GST-fusion proteins in 1 ml B88 (see above) containing 1% Triton X-100 for 2 h. After washing thoroughly bound proteins were eluted by 100 mM glycine, pH 2.54. 1 ml-fractions of both washing and elution steps were collected, elution fractions were neutralized with 50 µl of 1.5 M Tris (pH 8.8) solution and subsequently analysed by SDS-PAGE and Coomassie staining.

## Claims

1. A non-natural polypeptide selected from a pair of polypeptides, wherein one polypeptide comprises a sequence with at least 80 % identity to SEQ ID NO:1 derived from Dsllp, and an other polypeptide comprises a sequence with at least 80 % identity to SEQ ID NO:2 derived from δ-COP, wherein the two polypeptides are capable of binding to one another in an aqueous solution of 200 mM NaCl.

2. The non-natural polypeptide of claim 1, comprising a covalent modification, particularly wherein the covalent modification is a fluorophore, a chromophore, a radio label, an enzyme or an otherwise detectable moiety.

3. A nucleic acid encoding the non-natural polypeptide according to claim 1.

4. An affinity matrix comprising the non-natural polypeptide according to any of claims 1 or 2, and a solid support.

5. The affinity matrix of claim 4, wherein said polypeptide comprises SEQ ID NO:2, particularly wherein the polypeptide comprises less than 30 amino acids, more particularly less than 20 amino acids, particularly wherein the polypeptide is SEQ ID NO:2; or wherein the polypeptide comprises SEQ ID NO:1, particularly wherein the polypeptide comprises less than 50 amino acids, more particularly less than 40 amino acids, most particularly wherein the polypeptide is SEQ ID NO:1.

6. The affinity matrix of claims 4 or 5, wherein the solid support has a particle size of between 0,1 µm and 1 mm.

7. The affinity matrix of any of claims 4 to 6, wherein the polypeptide is coupled covalently or non-covalently to the solid support.

8. A kit comprising an affinity matrix according to any of claims 4 to 7, and a container, preferably also comprising an eluent.

9. Use of an affinity matrix according to any of claims 4 to 7 for the purification of a polypeptide according to any of claims 1 or 2.

10. Use of claim 9, wherein the polypeptide is expressed in a bacterium, a fungus or a higher eukaryotic cell, particularly wherein the polypeptide is expressed in *E.* coli, Bacillus, yeast, plant cells, insect cells or mammalian cells.

11. Use of a first polypeptide according to any one of claims 1 or 2 for the detection of a second polypeptide according to any one of claims 1 or 2.

12. Use of claim 11, wherein the polypeptide to be detected comprises a sequence with at least 80 % identity to SEQ ID NO:1, particularly SEQ ID NO:1, and the polypeptide to be used for detection comprises a sequence with at least 80 % identity to SEQ ID NO:2, particularly SEQ ID NO:2; or wherein the polypeptide to be detected comprises a sequence with at least 80 % identity to SEQ ID NO:2, particularly SEQ ID NO:2, and the polypeptide to be used for detection comprises a sequence with at least 80 % identity to SEQ ID NO:1, particularly SEQ ID NO:1.

13. Use of claims 11 or 12, wherein the polypeptide used for detection is a modified polypeptide according to claim 2.

14. Use of a first polypeptide according to any one of claims 1 or 2 for the dimerization with a second polypeptide according to any one of claims 1 or 2.

15. Use of claim 14, wherein the first polypeptide comprises a sequence with at least 80 % identity to SEQ ID NO:1, particularly SEQ ID NO:1, and the second polypeptide comprises a sequence with at least 80 % identity to SEQ ID NO:2, particularly SEQ ID NO:2; or wherein the first polypeptide comprises a sequence with at least 80 % identity to SEQ ID NO:2, particularly SEQ ID NO:2, and the second polypeptide comprises a sequence with at least 80 % identity to SEQ ID NO:1, particularly SEQ ID NO:1.

16. Use of claims 14 or 15, wherein dimerization occurs within a living cell.

17. Use of claim 16, wherein dimerization controls the biological activity of a polypeptide.

18. Use of claim 17, wherein the first polypeptide changes its cellular localization after addition of the second polypeptide.

19. Use of claims 16 to 18, wherein the second polypeptide is administered as a cell-permeable peptide, a polypeptide capable of being endocytosed, a polypeptide in a liposomal composition, or by induced transcription.

20. Use of claim 17, wherein the first polypeptide is rendered non-functional by addition of the second polypeptide.

21. Use of claim 20, wherein the first polypeptide is degraded or inhibited after addition of the second polypeptide.

22. Method for the purification of a polypeptide according to claims 1 or 2 comprising the following steps:
a) Contacting a solution containing said polypeptide with an affinity matrix according to any of claims 4 to 7, wherein the polypeptide of the affinity matrix is the other polypeptide of the pair of polypeptides according to claim 1;
b) Washing the affinity matrix; and
c) Eluting the bound polypeptide.

23. The method of claim 22, wherein step b) is a wash with an aqueous solution comprising more than 150 mM NaCl, particularly more than 250 mM NaCl and more particularly about 500 mM NaCl.

24. The method of claim 22 or 23, wherein step b) is a wash with with an aqueous solution comprising more than 0,05% non-ionic detergent, particularly more than 0,2% non-ionic detergent, more particularly 1% Triton.

25. The method of claims 22 to 24, wherein step c) is elution by an aqueous solution of 100 mM Glycin or 1 M NaCl.

26. The method of claims 22 to 25, wherein after step c) the pH of the eluate is adjusted to between pH 4-9, more preferably pH 5-8, even more preferably pH 6-8.


SEQUENCE LISTING

<110>  Garching Innovation GmbH

<120>  A pair of polypeptides with specific and strong interaction

<130>  G38374EP

<160>  2

<170>  PatentIn version 3.1

<210>  1

<211>  35

<212>  PRT

<213>  Artificial Sequence

<220>

<223>  amino acids 406 to 440 of the S. cerevisiae Dsl1 protein (D-type
       polypeptide)

<400>  1

Ala Val Ser Lys Asp Asp Asp Trp Asn Trp Glu Val Glu Asp Asp Asp
1               5                   10                  15

Ala Asp Ala Trp Gly Asp Glu Ile Asp Val Asn Ile Asp Asp Glu Glu
            20                  25                  30

Glu Lys Thr
        35

<210> 2

<211> 13

<212> PRT

<213> Artificial Sequence

<220>

<223> amino acids 242 to 254 of the S. cerevisiae Ret2 protein (R-type
      polypeptide)

<400> 2

Arg Ala Gly His Ser Ala Thr Gly Gly Met Lys Leu Gly
1               5                   10